# EUROPEAN PATENT APPLICATION

(11) **EP 1 336 393 A2**
(43) Date of publication of application: **20.08.2003**
(21) Application number: 03003346.8
(22) Date of filing: 13.02.2003
(51) Int. Cl.: A61F 2/06

(54) **Stent-prosthesis, delivery device and delivery set for stent-prosthesis**

(30) Priority: 14.02.2002 EP 02003467
(71) Applicant: Geis, John S., 82031 Grünwald (DE); Braun, Michael, 71522 Backnang (DE)
(72) Inventor: Geis, John S., 82031 Grünwald (DE); Braun, Michael, 71522 Backnang (DE)
(74) Representative: Cullinane, Marietta Bettina

(57) **Abstract**

The present invention relates to a stent-prosthesis (1), in particular an aortastent-prosthesis, comprising an essentially cylindrical tube (12) and at least one spring element (11, 11', 11") for attaching at least a part of the stent-prosthesis (1) to the inner wall of a blood vessel, wherein the tube (12) is made of flexible material, having a proximal end (121) and having at least a first and a second section (2, 3) being axially adjacent to each other, wherein in the first section (3) of the tube (12) at least one of said spring elements (11, 11', 11") is provided and the the second section (2), adjacent to the first section (3), being free of spring elements (11, 11', 11") and only being formed by the flexible material of the tube (12) and wherein the second - section (2) extends to the proximal end (121) of the tube (12), and wherein the at least one spring element (11, 11, 11") can be compressed in the radial direction of the tube (12) and in the relaxed state keeps the tube (12) at least over the first section (3), in which it is provided, in an open state. The invention further relates to a delivery device for such a vessel support, in particular a catheter system, and to a delivery set.

## Description

The present invention relates to a vessel support, in particular a stent-prosthesis device such as an aorta-stent-prosthesis. The invention further relates to a delivery device for such a vessel support, in particular a catheter system, and to a delivery set.

In the treatment of vascular diseases, such as aneurysms and dissections, it is known to support the affected tissue with a stent, or to replace the diseased vessel segment with a prosthesis or graft. By those means the rupture of the vessel wall can be avoided. This is of specific importance for diseases of the aorta, since the rupture of the aorta vessel wall can lead to internal bleeding and thereby to death.

By implanting a prosthesis, also a dissection of the vessel, in particular of the bow of the aorta, can be treated. These prostheses are in general implanted by a surgical procedure. Herein the diseased vessel becomes accesible by placing a cut in the area of the breastbone and opening the thorax. Especially because of the duration of the invasive operation, which, due to the complexity of the process, can be up to several hours, the surgery is hard on the patient. After the diseased tissue of the bow of the aorta has been removed and has been replaced by a prosthesis, and the prosthesis has been sutured to the healthy tissue, the surgical procedure is considered to be completed. It has, however, been found, that, despite the diseased tissue having been excised, diseases, such as dissections, occur as a late effect of the original disease. These late effects occur on the tissue, to which the prosthesis had been sutured. The disease can advance over the thoracic aorta, which is also referred to as the descending aorta. If the disease reaches a certain extent another surgery might become necessary.

One object of the present invention is therefore to provide a vessel support, which allows the treatment of thoracic aneurysm and / or dissection in the same surgical procedure as the reconstruction or replacement of the bow of the aorta, in particular the ascending aorta. This vessel support should also allow for preventive treatment of late effects of a vascular disease, such as a dissection or an aneurysm of the bow of the aorta, without the need of excising healthy tissue during the first surgical procedure. The vessel support should furthermore be easy to manufacture and easy to insert into the lumen of the vessel to be treated.

Another object of the invention is therefore a delivery device and a delivery set for delivery of the vessel support to the site in the vessel.

The invention is based on the finding, that these objects can best be achieved by a or by using a vessel support which constitutes a combination of a stent and a prosthesis in one body and which only needs to be permanently attached to the vessel on one of its end, whereas the other end can be attached to the inner side of the blood vessel by a force, such as a spring force.

To achieve these and other objects, there is provided a stent-prosthesis, in particular an aorta-stent-prosthesis, comprising an essentially cylindrical tube and at least one spring element for attaching at least a part of the stent-prosthesis to the inner wall of a blood vessel, wherein the tube is made of flexible material, having a proximal end and having at least a first and a second section being axially adjacent to each other, wherein in the first section of the tube at least one of the spring elements is provided and the second section, adjacent to the first section, being free of spring elements and only being formed by the flexible material of the tube and wherein said second section extends to the proximal end of the tube, and wherein the at least one spring element can be compressed in the radial direction of the tube and in the relaxed state keeps the tube at least over the first section, in which it is provided, in an open state.

The first section of the tube being free of spring elements and extending to the proximal end of the tube will hereinafter also be referred to as the "free section" or the prosthesis part. This part serves as a graft or prosthesis, i.e. it takes over the support and guide function of an excised part of a blood vessel. The prosthesis part can in particular replace an excised part of the aorta, preferably the bow of the aorta. The second section of the tube, in which at least one spring element is provided, will hereinafter also be referred to as the "resilient section" or the stent part. This part serves for supporting at least a section of the blood vessel into which it is inserted, in particular the thoracic aorta, and also serves for the attachment or fixation of the stent-graft to the blood vessel.

Preferably the diameter of the tube is essentially constant over its length in the implanted state. In a state, where the stent-prosthesis is not implanted in a blood vessel, but the spring elements are in a relaxed state, the diameter of the tube in the free section can be smaller than the diameter of the tube in the resilient section. This tapering of the tube is also referred to as oversizing the tube in the resilient section. In particular in that case the tube may be made by rolling a piece of material, so that two edges abut and a tapered tube is formed and subsequently sewing or otherwise joining the two edges of the material to each other. The diameter of the resilient part should in particular be larger than the diameter of the prosthetic part, when the stent-prosthesis is used as an aorta-stent-prosthesis. This is necessary, because the diameter of the resilient part should be oversized of about 2 to 8 mm to the diameter of the thoracic aorta into which it is to be inserted, in order to secure attachment.

Due to the structure of the inventive stent-prosthesis, one end of the tube can be sutured to a blood vessel wall or to another prosthesis and the other end can support itself on the inner side, in particular the inner circumference, of a healthy blood vessel by means of the at least one spring element. In this way the thoracic aorta can be supported and protected from the inside by the aorta-stent-prosthesis. This means that this part of the aorta does not need to be surgically removed during the surgery for the replacement of the bow of the aorta.

In addition, the inventive structure of the aorta-stent-prosthesis has the advantage that it can be inserted into the blood vessel in an easy way and reliably remains in the inserted position. This is ensured by the at least one spring element on the one hand and on the other hand by the possibility of suturing or otherwise surgically joining the other end to the blood vessel or a prosthesis for the bow of the aorta. For inserting and placing the aorta-stent-prosthesis in the blood vessel, in particular in the thoracic aorta, the section of the tube being free from spring elements, i.e. the prosthesis-part can be everted and tucked in, respectively, in the other section of the tube, i.e. the stent part of the aorta-stent-prosthesis. This is only possible, since the prosthesis part does not have any spring elements or other support means, but is only made of the flexible material of which the tube is manufactured. By tucking one section of the aorta-stent-prosthesis into the other, the overall length of the aorta-stent-prosthesis is considerably reduced for the insertion and thereby the insertion and placing of the aorta-stent-prosthesis is facilitated. Also a small operation site is thereby provided. Once the stent part has been positioned in the blood vessel and the spring elements have expanded, the prosthesis part of the stent-prosthesis can be pulled out of the resilient part. This unfolding or pulling out of the free section can be accomplished by the use of appropriate tools, such as tweezers. The preferred tool will be later described with respect to the delivery device.

Finally, any potentially existing difference in diameter of the prosthesis or blood vessel, to which the aorta-stent-prosthesis is to be sutured, and the diameter of the tube can be compensated by the flexible material of the tube without the risk of rupturing the tube. This is in particular true if the prosthesis part of the aorta-stent-prosthesis is long. The length of the resilient part is chosen to be sufficient to receive the free part and to secure the stent part against the inner surface of a blood vessel.

The provision of the free section, which extends to the proximal end of the tube, also entails the further advantage that the placing of the aorta-stent-prosthesis in the blood vessel, in particular the placing of the stent part in the blood vessel, does not have to be carried out with a high degree of precision. The prosthesis part of the aorta-stent-prosthesis is preferably designed to be longer than the section of the blood vessel, which is to be replaced. Thereby, even in the event of a slight misplacement of the stent part in the blood vessel, the length of the vessel part, which is to be replaced, can safely be covered. If the stent part is placed too close to the end of the blood vessel, created by the excision, the overhang at the prosthesis part can be cut off after the stent part has been placed and before the prosthesis part is being sutured to the other end of the blood vessel or to another prosthesis. Thereby, insertion and placing of the aorta-stent-prosthesis is simplified and the duration of the surgical operation can be reduced. The ends of the blood vessel referred to in this context are preferably the openings formed by the excision of diseased tissue.

It should be noted that the first and second section of the stent-prosthesis do not form separate parts, but are areas of one device.

The term distal end of the stent-prosthesis refers to the end, which during insertion is the farthest from the point of entry into the blood vessel. At the same time the distal end according to the definition of the invention is preferably the end of the stent-prosthesis, which, in the inserted state, is the farthest from the heart.

The term proximal end of the stent-prosthesis refers to the end, which during insertion is the closest to the point of entry into the blood vessel. At the same time the proximal end according to definition of the invention is preferably the end of the stent-prosthesis, which, in the inserted state, is the closest to the heart.

In one embodiment the second section being free of spring elements extends from the proximal end of the tube over at least one third of the length of the tube. In this context the length of the tube is the overall length of all sections in the state before the one section is tucked into the other and before a potential cutting of the tube after insertion. Preferably the section free of spring elements can even extend over at least half of the length of the tube. By this partitioning of the tube into the free section and the resilient section with the preferred lengths, the length of the stent-prosthesis during insertion can be minimized by tucking the free section into the resilient section. At the same time, however, a sufficient stability of the attachment or fixation of the section having spring elements to the inner side, in particular the inner circumference, of the blood vessel can be provided.

The at least one spring element preferably extends over the entire circumference of the tube. Preferably the spring element, however, has a limited extension in the axial direction of the tube. In case of a spring element in the form of a ring, the axially limited area of the tube is defined by the thickness of the material of which the ring is made. By this embodiment a uniform radial contraction and expansion of the tube can be ensured. According to the present invention the spring elements only have a radial extension over the outer diameter of the tube to the extent of their thickness but no additional extension is provided. This means that the spring elements are in contact with the tube over their entire length in both the relaxed and compressed state.

In a preferred embodiment the at least one spring element is designed for attaching at least the first section of the tube to the entire inner circumference of a vessel wall. This feature can lead to a secure end-to-end connection of the stent-prosthesis and the blood vessel.

Preferably the at least one spring element is attached to the outside of the tube. By this arrangement of the spring elements, several advantages can be achieved. By choosing a suitable material of the spring elements it can be achieved that these spring elements can grow into the tissue of the blood vessel. The stability of the connection between the stent-prosthesis and the blood vessel can thereby be improved. Furthermore turbulences, which might occur, when the spring elements are attached to the inside of the tube can be avoided. Finally the inner lumen of the stent-prosthesis is not or only to a minimal extent decreased by the spring elements. The spring elements, which are attached to the outside surface of the tube force themselves onto and potentially slightly into the vessel wall, whereby the tube may be pulled outwards in the radial direction at those locations. The inner lumen might thereby be increased at those locations, but definitely not decreased. Thereby, the diameter of the tube will be essentially uniform over its length in the implanted state, even in the vicinity of the position at which the at least one spring element is attached. Furthermore, when the spring elements are attached on the outside of the tube, the free section of the tube may be everted or tucked into the resilient section for the insertion of the stent-prosthesis and may be pulled out of the resilient section, without the risk of the free section getting caught by one or more of the spring elements.

It is in particular preferred to attach the at least one spring element to the outside of the tube by sewing. This manner of attachment can prevent a movement of the spring elements on the tube and at the same time does not present risks for the health of the patient. In addition this manner of attachment allows the spring elements to be received in the vessel tissue and to potentially grow into the vessel tissue.

In one embodiment the at least one spring element is a spring ring. According to the present invention the at least one spring element can have various different shapes and can be made of different materials. It is, however, preferred to use a spring elements, which has a springiness, by which it can keep the tube in an open state, when the spring is in the released state. For this reason a spring ring is preferred. This spring ring preferably has an inner diameter in the relaxed state, which is essentially equal to the outer diameter of the tube. The diameter of the ring can be reduced by applying pressure on its outer circumference, i.e. it can be compressed towards the axis of the tube. When the pressure is removed the spring ring can expand and can keep the tube in its essentially cylindrical shape, at least in the section where the spring elements are provided.

In a preferred embodiment the first section of the tube, wherein the at least one spring element is provided, extends to the distal end of the tube. This means that at the end of the tube, which is opposed to the free section, a spring element is provided. Thereby it can be prevented, that flexible material extends over this last spring element and potentially hinders or disturbs the blood flow.

The number of spring elements, which are provided in the resilient section of the tube, can be for example at least three, preferably at least four and in particular preferred at least five.

The inventive vessel support is preferably an aorta-stent-prosthesis. This use of the inventive vessel support allows for the best use of the advantages achievable with the vessel support. After the replacement of a diseased bow of the aorta by prosthesis, the blood pressure acting on the thoracic bow of the aorta and the upper area of the descending aorta increases. The inventive aorta-stent-prosthesis can ideally act as a preventive means for healthy tissue or as support means for tissue, which is already diseased. In particular the advantage of the inventive aorta-stent-prosthesis, namely that this combines a prosthesis and a vessel stent in an axial arrangement, can be ideally used in this application. The stent part of the aorta-stent-prosthesis can act together with the vessel wall by means of frictional connection while the prosthesis part of the aorta-stent-prosthesis can be sutured to the vessel wall.

In one embodiment the tube has at least one aperture in the tube wall, wherein the at least one aperture leads into the lumen of a side tube attached to tube. By this arrangement, at least a part of the bow of the aorta, including one or more branches from the bow, can be reconstructed by the aorta-stent-prosthesis. Thereby there is no necessity to provide a separate prosthesis for the bow to be used together with an embodiment of the inventive aorta-stent-prosthesis. The duration of the surgery can thereby be further decreased. The side tubes attached to the openings in the tube walls are designed to have a diameter of essentially the same size as the diameter of the branches of the bow of the aorta and are in general of a smaller diameter than the main tube. The side tubes may be sewed to the tube.

In a preferred embodiment the section being free of spring elements is a prosthesis in the shape of the bow of the aorta, including at least parts of all its branches. The side tubes of the prosthesis are connected to apertures in the tube wall of the main cylindrical tube and are for example arranged so that they may be connected by suturing or other surgical connections to the brachiocephalic trunc, left common carotid artery and/or left subclavian artery.

The present invention further relates to a delivery device for placing a stent-prosthesis in a blood vessel, preferably in the thoracic aorta of a human body, wherein the delivery device comprises a sleeve, having at least one lumen for receiving a stent-prosthesis, wherein in said lumen an attachment device being movable in the longitudinal direction of the sleeve is provided, wherein the attachment device is designed to be firmly attached to one end of the stent-prosthesis, which end is free from spring elements and is only formed of the flexible material of the tube. The sleeve of the device is preferably a catheter. The position at which the stent-prosthesis can be placed with the inventive device can be in the aorta, preferably at a position closer to the heard as the renal arteries.

The attachment device is preferably designed for non-detachable connection to the end of the tube, where the free section is located. Due to the strong connection that can be realized between the attachment device and the aorta-stent-prosthesis it becomes possible to pull the prosthesis part of the aorta-stent-prosthesis out of the stent part after placing the stent part at the desired position and allowing fixation of the stent part by means of the spring. elements. The longitudinally movable attachment device is therefore preferably designed to be a pull device. For this purpose the attachment device is preferably connected or part of a longitudinal tubular element, which is accommodated in the lumen of the catheter. This tubular element is preferably of a sufficient stability, in particular against bending and kinking, so that during the positioning and implementation of the aorta-stent-prosthesis in the blood vessel, the tubular element together with the attachment device can stop the aorta-stent-prosthesis, in particular the stent part from moving within the catheter towards the proximal end of the catheter. The proximal end of the catheter is considered to be the end, which is the closest to the surgeon, when inserting the stent-prosthesis via the catheter.

Preferably the delivery system further comprises a balloon. In that case the attachment device preferably has an aperture through which the balloon can be moved, when the balloon is in a deflated state. This arrangement allows for the deflated balloon to be maintained in a position spaced from the aorta-stent-prosthesis in the axial direction of the catheter when the aorta-stent-prosthesis is inserted. After the aorta-stent-prosthesis is positioned, however, the balloon can be brought into a position within the stent part of the aorta-stent-prosthesis. If the balloon is dilated in the latter position, it can be used to press the tube and spring elements against the vessel walls and, if necessary, to widen the vessel lumen. While the aorta-stent-prosthesis is inserted and the free section is pulled out of the resilient section, the balloon, due to its position spaced apart from the position of the aorta-stent-prosthesis, does not hinder the necessary movements of the tube. In addition the diameter of the sleeve or catheter can be decreased due to this arrangement, since the balloon catheter will only enter the area of the catheter where the aorta-stent-prosthesis is located, after the spring elements have been deployed.

The invention further relates to a delivery set for delivering an stent-prosthesis to the lumen of a blood vessel comprising at least one catheter having at least one lumen and a stent-prosthesis being accommodated in the lumen, the stent-prosthesis having a first section having spring elements at its distal end and a second section being free from spring elements at its proximal end, wherein the second section is everted into the first section, with the proximal end of the stent-prosthesis being accessible from the proximal end of the first section and being firmly attached to an attachment device being provided in the lumen of the catheter. This set, which might be prepackaged allow the surgeon to perform the surgery in a comparatively short amount of time.

The stent-prosthesis according to the invention can advantageously be used in a method for simultaneous replacement of at least a part of the bow of the aorta and preventive treatment of at least a part of the thoracic aorta, comprising the steps of excising at least a part of the bow of the aorta, introducing a delivery device being loaded with an aorta-stent-prosthesis having a stent part and a prosthesis part into the thus formed opening of the thoracic aorta, deploying at least the stent part of the aorta-stent-prosthesis into the thoracic aorta, deploying the prosthesis part of the aorta-stent-prosthesis from the delivery device detaching the aorta-stent-prosthesis from the delivery device, removing the delivery device, and surgically joining the prosthesis part of the aorta-stent-prosthesis to the ascending aorta or remaining part of the bow of the aorta. Preferably the stent-prosthesis is provided in the delivery device in a state, where the prosthesis part is everted into the stent part and the step of pulling the prosthesis part out of the stent part at a point during the procedure after at least a part of the stent part has been deployed.

The stent-prosthesis can be used in a method for delivering an aorta-stent-prosthesis to the thoracic aorta, wherein the method comprises the steps of introducing a delivery device comprising at least one outer catheter into the thoracic aorta from the direction of the ascending aorta, pulling back the outer catheter, thereby deploying at least a part of a section of the aorta-stent-prosthesis having spring elements, moving an attachment device guided in the outer catheter and attached to one end of the aorta-stent-prosthesis opposite to the end having spring elements in the direction away from the aorta-stent-prosthesis, thereby pulling the section of the aorta-stent-prosthesis being free from spring elements out of the section of the aorta-stent-prosthesis and separating the aorta-stent-prosthesis device from the attachment device. The free section of the stent-prosthesis and thereby the hole prosthesis is preferably detached from the attachment device in the vicinity of the attachment device.

The aorta-stent-prosthesis may be delivered by a method, where in addition a balloon catheter is guided through an aperture in the attachment device to the distal end of the aorta-stent-prosthesis after the deployment of the section of the aorta-stent-prosthesis having spring elements, the balloon is dilated, the balloon catheter is pulled back over at least the section of the aorta-stent-prosthesis having spring elements and thereby the aorta-stent-prosthesis is unraveled and fully opened.

The features and advantages that were described with respect to the stent-prosthesis, the delivery system and the delivery set also hold good, as far as applicable, for the respective other objects and vice versa.

The invention will now be explained in more detail with reference to examples shown in the enclosed figures, wherein:
Figure 1: is a schematic representation of one embodiment of the inventive stent-prosthesis;
Figure 2: is a schematic representation of the embodiment of figure 1 embodiment of the inventive stent-prosthesis in an everted state;
Figure 3: is a schematic representation of one embodiment.of the inventive stent-prosthesis in a twice-everted state;
Figures 4a- 4c: are schematic representations of the inventive delivery set in different situations during the inserting and deploying of the stent-prosthesis;
Figure 5: is a schematic representation of an aorta-stent-prosthesis in the state implanted in the vessel;
Figure 6a and Figure 6b: are schematic representations of another embodiment of the aorta-stent-prosthesis according to the invention, where the prosthesis part is in the shape of the bow of the aorta, with branches.

The representations are not to scale.

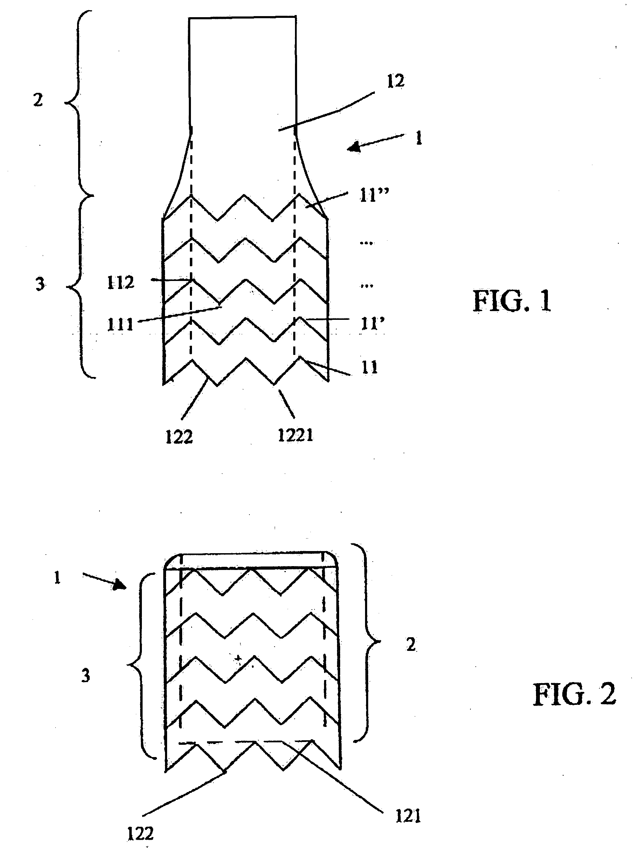

In figure 1 a schematic representation of the aorta-stent-prosthesis 1 according to one embodiment of the invention is shown. A tube 12 of flexible material forms the body of the aorta-stent-prosthesis 1. This tube has a proximal end 121 and a distal end 122. In the embodiment shown in Figure 1, tips 1221 of the tube 12 form the distal end 122. The tips 1221 are equidistantly spaced arranged over the circumference of the distal end 122 of the tube 12. The proximal end 121 is a straight end.

A spring element 11 is guided along the shape of the tips 1221 at the distal end 122 and is attached there. Preferably this spring element 11 is sewed to the outside of the tube 12. It lies, however, also within the scope of the invention to attach the spring element 11 at the distal edge of the tube 12 along the shape of the tips 1221. In the embodiment shown in Figure 1 further spring elements 11' to 11" are provided in an equally spaced fashion on the tube 12 towards the proximal end 121 of the tube 12. These spring elements 11' through 11" have the same shape as the spring element 11 located on the distal edge of the tube 12. The spring elements 11' through 11" form closed loops or rings around the circumference of the tube 12, which have a zigzag-shape, with the tips and valleys of the zigzag pointing in the longitudinal direction of the tube 12. In the embodiment shown in figure 1 the spring elements 11, 11' through 11" are arranged on the tube 12 in such a manner that the tips 112 and valleys 111 of the respective spring elements 11, 11' through 11" are aligned on lines parallel to the longitudinal axis of the tube 12 with the tips and valleys respectively of the further spring elements.

Thereby two sections 2 and 3 are formed along the tube 12, which sections 2 and 3 are adjacent to each other along the length of the tube 12. The section 3 extends from the distal end 122 of the tube 12 up to the tips 112 of the spring element 11" spaced the furthest apart there from, i.e. in figure 1 the upper spring element 11". This section 3, which is hereinafter also referred to as the resilient section or the stent part, is followed by the section 2, which is free from spring elements and will hereinafter be referred to as the free section or the prosthesis part. The section 2 ends at the proximal end of the tube 12. In the free section 2 no spring elements or other support or reinforcement means are provided. This section 2 is rather formed by the flexible material of the tube 12 exclusively.

Figure 1 shows the embodiment of the stent-prosthesis in a state, where the spring elements are completely relaxed, i.e. have reached their maximal diameter. In this state, the section 2 of this embodiment has a smaller diameter than the section 3. This oversizing of section 3 can be advantageously used to allow for a higher radial force to be applied by the spring elements, once the stent-prosthesis is implanted in a blood vessel. The shape of the stent-prosthesis in the implanted state is,indicated by the dashed lines. In that state the diameter of the overall stent-prosthesis is approximately constant over the hole length of the tube.

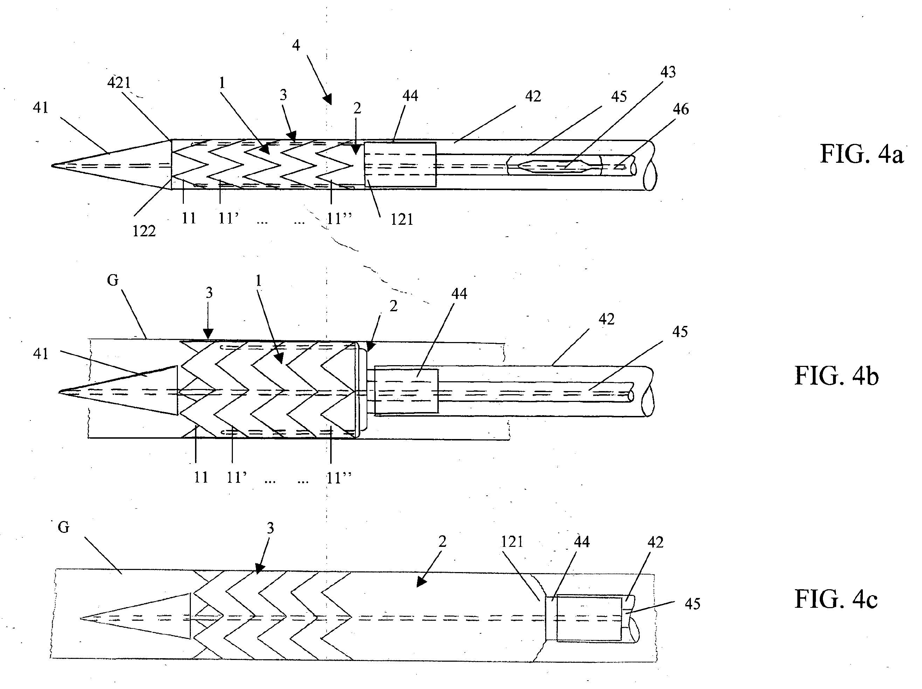

In figure 2 the aorta-stent-prosthesis 1 from figure 1 is schematically shown in an everted or tucked in state. Herein the section 2 is everted into section 3. In this state the aorta-stent-prosthesis can be placed into a delivery system such as a delivery catheter, one embodiment of which will be described later in detail with reference to figures 4a through 4c.

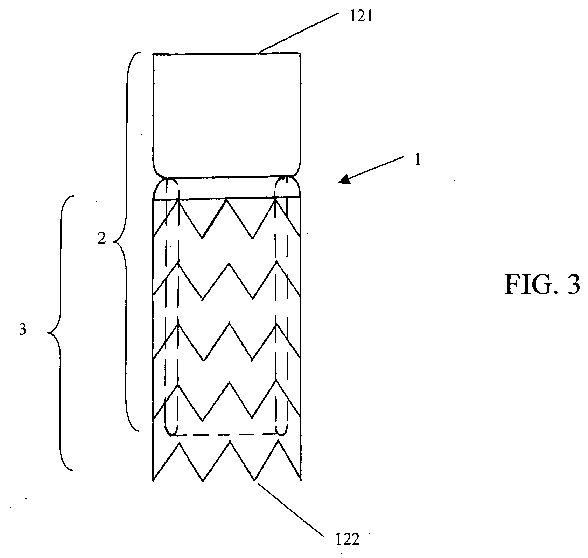

In figure 3 a further preferred possibility of eversion of the free section is illustrated. In that case the free section is twice everted and folded, respectively. The access to the proximal end 121 of the tube is easier in this embodiment and the pulling of the free section out of the resilient section is facilitated. The length over which the free section 2 in the folded state extends into the resilient section 3, depends on the overall length of the free section and on the attachment device to be used for attaching the proximal end 121. Preferably the free section 2 is essentially completely received in the resilient section 3 with only a minor amount of the free section at the proximal end 121 projecting over the resilient section 3. In order to be able to keep the diameter of the thus folded and everted aorta-stent-prosthesis 1 to a minimum, which is of relevance for the inserting the aorta-stent-prosthesis 1 into a blood vessel, it is essential, that the free section does not have any spring elements or other support or reinforcement means, but is exclusively formed by the flexible material of the tube 12.

In figures 4a through 4c a delivery system including an inventive aorta-stent-prosthesis, which together form an inventive delivery set is shown. Figure 4a shows the delivery system 4 with the aorta-stent-prosthesis 1 accommodated therein in the state, in which the delivery system can be inserted in the blood vessel.

The delivery system 4 comprises in the embodiment shown in Figure 4 a an essentially cylindrical sleeve 42, which on one end 421 is closed by a guide tip 41. The guide tip 41 is movable via a guide wire (not shown). This guide wire is guided in a guide wire lumen 46, which extends over the entire length of the sleeve 42.

In the sleeve 42 an attachment device 44 for attachment of the aorta-stent-prosthesis 1 is provided and can be moved in a longitudinal direction therein. This attachment device 44 is for example a cylinder. In the radial center of the attachment device 44 an aperture for the guide wire 43 is provided. At the one end of the attachment device 44, which is opposed to the proximal end of the sleeve 42 the attachment device 44 is connected to a guide sleeve 45, which is guided in the sleeve 42.

In the area between the attachment device 44 and the distal end of the guide sleeve 45 a balloon 43 is indicated. The balloon 43 can be moved and dilated by means of a balloon catheter (not shown). Hence a balloon catheter can be included in the delivery system 4. The balloon catheter can slide on the guide wire lumen 46 and can run within the guide sleeve 45. Preferably the balloon 43 of the balloon catheter is in the position as shown in Figure 4a, i.e. viewed from the guide tip 41 behind the attachment device 44.

At the proximal ends of the sleeve, of the guide sleeve, of the balloon catheter and of the guide wire actuating means can be provided, which allow the movement of these components independently from one another. These actuating means are not shown in figure 4.

The aorta-stent-prosthesis 1 is arranged in the delivery system 4 so that the distal end 122 thereof lies close to the distal end 421 of the sleeve 42. In the section 3 adjacent to the distal end 122 of the aorta-stent-prosthesis 1, wherein spring elements 11, 11' through 11" are provided, the free section 2 is received. In the shown embodiment the free section 2 is inserted into the resilient section 3 in a double-folded state. The free section 2 therefore runs, starting from the last spring element 11" first in the direction of the distal end 122 of the aorta-stent-prosthesis 1 and is then folded, so that it continues in the direction, which is opposed to the distal end 122 of the aorta-stent-prosthesis 1. At the proximal end 121, which terminates the free section 2, the aorta-stent-prosthesis 1 is attached to the attachment device 44.

This attachment can be realized in several ways. Preferably the attachment method however leads to a non-detachable connection, so that the aorta-stent-prosthesis 1 can only be separated from the attachment device 44 by means of cutting devices, such as scissors. Other methods of attaching may be adhering, sewing, welding. Also attachment via clamping the proximal end 121 in a groove provided in the attachment device may be considered or the provision of a magnetic system, such as a ring acting together with a magnet attached to the tube, can be chosen. In the latter mentioned options of clamping or the use of magnets, however, additional fixation, for example by adhering or welding should be carried out.

In order to be capable of producing such a connection the attachment device is preferably designed to be for example fusible or weldable. This characteristic can be achieved by using a material with this property for the whole attachment device or by providing a layer on the outside, the front side or in any recesses created in the attachment device, where the end of the stent-prosthesis 1 will be received. It is also possible to apply a layer of material, which allows the flexible material to be sewed to on the outside of the attachment device. Alternatively the attachment device could be made of such a material. Furthermore it is possible to provide holes in the attachment device to allow suture used to attach the prosthesis to the device to be guided through those holes.

The strong connection between the aorta-stent-prosthesis and the attachment device 44 is necessary in order to be able to pull the free section 2 out of the resilient section 3, without risking the connection to fail.

In figure 4b there is shown the state, which the delivery system 4 and the aorta-stent-prosthesis 1 adopt, when the aorta-stent-prosthesis 1 is to be deployed from the delivery system 4. Herein the sleeve 42 is pulled back along its longitudinal axis, i.e. moved in the direction of its proximal end. Thereby the guide tip 41 separates from the distal opening of the sleeve 42. The guide sleeve 45 and the attachment device 44 connected thereto as well as the aorta-stent-prosthesis 1 do not change their positions in the longitudinal direction. However, the spring elements 11, 11' through 11", which had been reduced to a smaller diameter as their relaxed diameter by the sleeve 42, spring open and get in contact with the vessel wall (G). It is also possible that the attachment device 44 is designed to have such a diameter that it abuts to the inner surface of the sleeve 42 and during retraction of the sleeve 42 serves as a detent for the resilient section 3. It is also possible, that attachment device 44 is used as a pusher to push the resilient section 3 out of the sleeve 42. Once the whole resilient section 3 has been released from the sleeve 42, that means also the last spring element has left the sleeve 42, the free section 2, accommodated in the resilient section 3 in a folded fashion, can be pulled out.

This is indicated in figure 4c. For this purpose the guide sleeve 45, which is connected via the attachment device 44 to the proximal end 121 of the aorta-stent-prosthesis 1 is moved in the direction of the proximal end of the sleeve 42. Thereby the free section 2 is pulled out of the resilient section 3. It is also possible to simultaneously move the guide sleeve 45 and the sleeve 42 in the direction of their proximal ends. In that case the free section will be pulled out of the resilient section at the same time as the resilient section is being deployed from the sleeve.

Once the aorta-stent-prosthesis 1 has adopted the stretched state as shown in 4c, the deployment procedure is generally completed. If a balloon 43 is provided in the delivery system 4 the balloon can now be moved forward to the distal end 122 of the aorta-stent-prosthesis 1 and can there be dilated and expanded, respectively. Starting from this end 122 each area of the tube 12, including both sections 2 and 3 are opened and the section 3 is in addition simultaneously brought in close contact with the vessel wall G. The aorta-stent-prosthesis 1 can then be separated from the attachment device 44 and the delivery system 4 can be removed from the aorta-stent-prosthesis 1 and from the vessel. Depending on the attachment method chosen for the connection between the attachment device 44 and the aorta-stent-prosthesis 1, the connection can be released by mechanically, physically or chemically removing adhesive material used, or the aorta-stent-prosthesis 1 can be cut off in the vicinity of the attachment device 44. In the latter case it can be ensured that the aorta-stent-prosthesis 1 has the correct length and there is no risk that residues of the attachment mechanism, such as suture or adhesive material enters into the blood vessel.

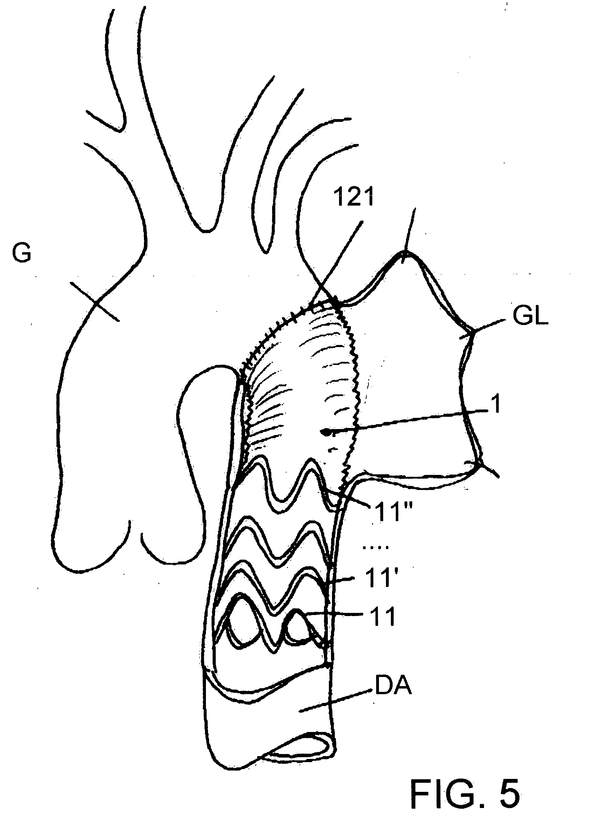

In figure 5 a vessel (here the thoracic aorta with the bow of the aorta) is shown with the aorta-stent-prosthesis 1 according to one embodiment of the invention having been implemented. The proximal end 121 of the aorta-stent-prosthesis 1 is sutured or connected by other surgical connection means, such as stapling, to the vessel wall G in the area of the bow of the aorta. The distal end 122 of the aorta-stent-prosthesis 1 abuts to the inner side of the descendant thoracic aorta (DA) with the spring elements 11, 11' through 11" and the outside of the tube 12 in the resilient section 3.

It should be noted that in figure 5 the wall of the thoracic aorta is not shown in the area of the spring elements to allow visibility of these elements. It will, however, be understood that the spring elements are in contact with the inside of the thoracic aorta.

In surgeries for treatment of vascular diseases, it is possible to excise the diseased tissue and remove the hole part of the blood vessel, where the disease occurred. It is however also possible to open the blood vessel, e.g. by placing one cut in the longitudinal direction of the blood vessel and placing two cuts in the radial direction at the ends of the longitudinal cuts. Thereby the diseased tissue can be removed and the remaining tissue can be closed again to have the form of the blood vessel. The thus recreated blood vessel will, however, not be capable of fulfilling the functions of the original blood vessel. In figure 5 the remaining part (GL) of the tissue, which might be sutured to the vessel wall or to the stent prosthesis is indicated.

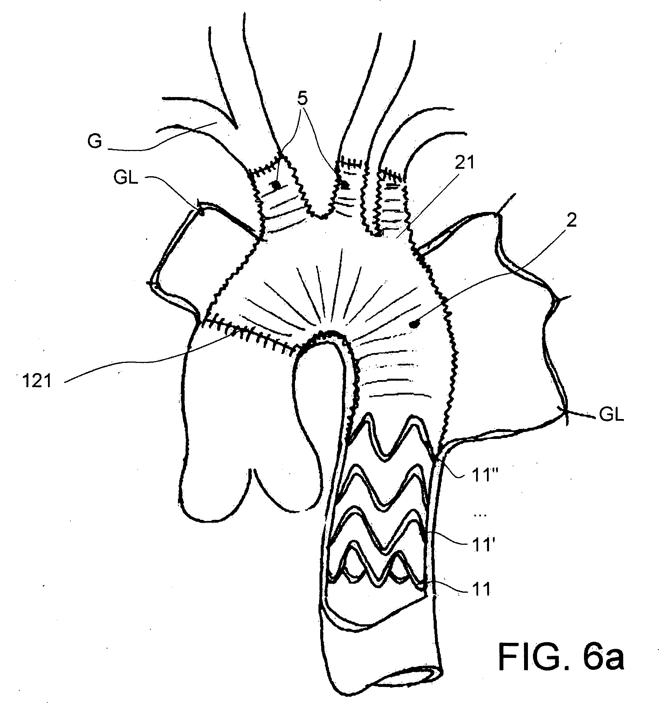

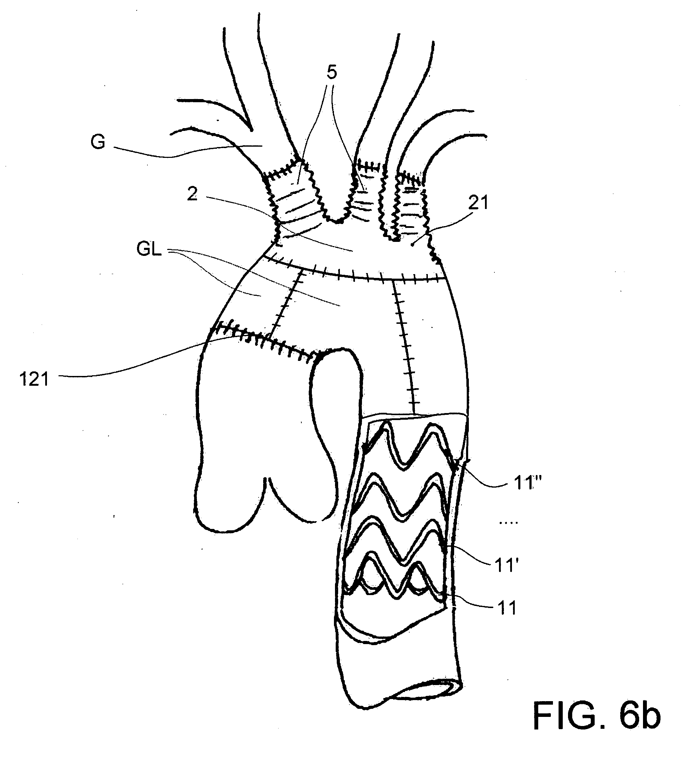

In figure 6a a further embodiment of the inventive stent-prosthesis is shown in the implanted state at the aorta. It should be noted that also in figure 6a and figure 6b the wall of the thoracic aorta is only partially shown to be able to see the spring elements in the thoracic aorta. The stent-prosthesis 1 has a first section 2 , wherein several apertures 21 are provided. To each aperture 21 a side tube 5 is sewed, to connect the lumen of the side tube 5 with the lumen of the first section 2 of the stent-prosthesis 1. The side tubes 5 are made of the same flexible material as the tube 12 of the stent-prosthesis 1. As shown in figure 6a the side tubes 5 can be used as prosthesis for at least a part of the branches of the aorta, which are previously cut away together with the bow of the aorta.

As can be derived from figure 6a the aorta had been cut open and the diseased parts of the bow of the aorta had been excised, whereas the remaining tissue (GL) was left in place. Once the stent-prosthesis has been implanted and especially the proximal end has been sewed to the ascending aorta, the remaining tissue can be sutured together, as shown in Figure 6b. This reconstruction of the bow of the aorta with tissue can be advantageous with respect to the blood flow in the tissue, i.e. the blood flow can be maintained. In addition the reconstructed bow of the aorta can provide an additional security means to keep in particular the stent part of the stent-prosthesis in place. The closing of the tissue around the stent-prosthesis leaves the stent-prosthesis in an interposed position.

With the embodiment of the stent-prosthesis as shown in figure 6a and 6b it will become possible-to replace the bow of the aorta including parts of its branches at the same time as implementing a stent section into the thoracic aorta for preventive purposes. The insertion of this stent-prosthesis will be achieved in the same manner as described with respect to Figures 4a to 4c. The attachment device 44 will in this embodiment preferably be connected to the main tube 12 of the stent-prosthesis 1, whereas the side tubes will not be connected to the attachment device 44. After the stent-prosthesis has been inserted, the second section has expanded in the blood vessel and is attached via the spring elements, the proximal end 121 of the stent-prosthesis 1 will be sutured to the ascending aorta, while the side tubes 5 will be sutured or otherwise surgically joined to the branches of the aorta.

The embodiments described above are only examples of realizing the invention. It will be clear that the invention is not limited to these embodiments.

For example the number of spring elements can vary, depending on the size of the vessel to receive the stent section and depending on the length of the free section as well as additional criteria, such as the age and blood pressure of the patient. Also the spacing between the individual spring elements can be determined according to the intended use of the aorta-stent-prosthesis. For example up to fifteen spring elements in equidistant spacing of up to 5mm can be provided. Also the shape of the spring elements is not limited to the described zigzag-shape. As long as the spring element allows on one hand the reduction of the diameter of the aorta-stent-prosthesis to the inner diameter of the sleeve of a delivery system and on the other hand can open the tube to the desired size in the relaxed state, other spring elements can also be used. These include spring rings or loops, which have a wave form or a crown form in the axial direction.

Also the alignment of the spring elements is not limited to the illustrated alignment. When spring rings having a zigzag shape are used, the tips of one spring ring could for example face the tips of the adjacent spring ring.

It is also possible to provide connection elements between the individual spring elements, which can for example include a wire extending in the axial direction of the tube between the spring elements. By these means the length of the resilient section and the spacing between the spring elements can be maintained constant even during the insertion procedure and the resilient section is provided with a stability against twisting.

Also the length of the aorta-stent-prosthesis may be varied. For example an aorta-stent-prosthesis with an overall length of 25 to 30 cm can be produced. The length of the aorta-stent-prosthesis in the implanted state can then be adjusted by cutting, as described above. The length of the resilient area can for example be 5 to 15 cm over which the desired number of spring elements can be distributed.

The material for the aorta-stent-prosthesis and the delivery system can be chosen from all materials commonly used in the manufacturing of medical devices for the respective parts. The tube may be made of a polyester material, in particular polyester fiber material, such as Dracon material, since this material is very flexible and can therefore be easily tucked into the resilient section. On the other hand this material can be sutured and can be cut easily. The material for the spring element can for example be a Nitinol wire, a CoCr-alloy or a wire made of stainless steel. The spring element can consist of a helical spring, which is placed in a ring, loop or zigzag form.

The spring elements attached to the tube, can be adhered to the respective surface, inner or outer surface, they can be included in the flexible material of the tube, for example by weaving, or they can be sewed to one or both of the sides, inner or outer side of the tube. Herein the spring elements can be connected to the tube over their entire length by means of for example a continuous seam. It is however also possible to sew the spring elements to the tube only by means of individual stitches in greater distances over the length of the spring element.

It is also possible to design the distal end of the tube in a way that it forms a flat end, that means that there are no tips provided. In this case either zigzag shaped spring elements or a flat spring ring can be used at the end.

Even though the provision of only two sections, a resilient and a free section is preferable, it is also possible according to the present invention to provide for example three sections. In that case two free sections could be provided from the ends of the tube and the resilient section could be enclosed between the two free sections.

The sleeve of the delivery system, which is preferably a catheter, can have a constant diameter over its length. The sleeve can however also provide an area in the region of the distal end, where the diameter is larger compared to the diameter of the remaining length. The aorta-stent-prosthesis can be ideally received in this area with a larger diameter. The step formed from the transition of the larger to the smaller diameter of the sleeve can also serve as a detent for the aorta-stent-prosthesis during the deployment procedure.

The present invention therefore provides an aorta-stent-prosthesis, which can be implanted easily and in a short period, so the duration of a surgery can be reduced. The aorta-stent-prosthesis can in addition be secured in the implanted position and a preventive treatment of tissue diseases, such as dissections, becomes possible at the same time as a diseased part of the vessel can be replaced by a prosthesis. In addition the invention provides a delivery system for such an aorta-stent-prothesis, as well as a delivery set, which includes the aorta-stent-prosthesis and the delivery system, in which the aorta-stent-prothesis is loaded. The provision of such a set will decrease the time necessary for the implantation, since the surgeon or assistant does not have to prepare and load the aorta-stent-prosthesis into the delivery system at the site of the surgery. Also the risk of incorrect loading can be reduced.

## Claims

1. A stent-prosthesis (1), in particular an aorta-stent-prosthesis, comprising an essentially cylindrical tube (12) and at least one spring element (11, 11', 11") for attaching at least a part of the stent-prosthesis (1) to the inner wall of a blood vessel,
wherein the tube (12) is made of flexible material, having a proximal end (121) and having at least a first and a second section (2, 3) being axially adjacent to each other,
wherein in the first section (3) of the tube (12) at least one of said spring elements (11, 11', 11") is provided and the the second section (2), adjacent to the first section (3), being free of spring elements (11, 11', 11") and only being formed by the flexible material of the tube (12) and wherein the second section (2) extends to the proximal end (121) of the tube (12),
and wherein the at least one spring element (11, 11', 11") can be compressed in the radial direction of the tube (12) and in the relaxed state keeps the tube (12) at least over the first section (3), in which it is provided, in an open state.

2. A stent-prosthesis as claimed in claim 1, wherein the second section (2) being free of spring elements (11, 11', 11") extends from the proximal end (121) of the tube (12) over at least one third of the length of the tube (12).

3. A stent-prosthesis as claimed in claim 1 or 2, wherein the at least one spring element (11, 11', 11") extends over the entire circumference of the tube (12).

4. A stent-prosthesis as claimed in claim 1, 2 or 3, wherein the at least one spring element (11, 11', 11") is designed for attaching at least the first section (3) of the tube (12) to the entire inner circumference of a vessel wall.

5. A stent-prosthesis as claimed in any of claims 1 through 4, wherein the at least one spring element (11, 11', 11") is attached to the outside of the tube (12).

6. A stent-prosthesis as claimed in any of claims 1 through 5, wherein the at least one spring element (11, 11', 11") is sewed onto the outside of the tube (12).

7. A stent-prosthesis as claimed in any of claims 1 through 6, wherein the at least one spring element (11, 11', 11") is a spring ring.

8. A stent-prosthesis as claimed in any of claims 1 through 7, wherein the first section (3) extends to the distal end (122) of the tube (12).

9. A stent-prosthesis as claimed in any of claims 1 through 8, wherein at least five spring elements (11, 11', 11") are provided in the first section (3).

10. A stent-prosthesis as claimed in any of claims 1 through 9, wherein the tube (12) has at least one aperture in the tube wall, wherein the at least one aperture (21) leads into the lumen of a side tube (5) attached to tube (12).

11. A stent-prosthesis as claimed in claim 10, wherein the second section (2) is a prosthesis in the shape of the bow of the aorta, including at least part of its branches.

12. A delivery device for placing a stent-prosthesis in a blood vessel, preferably in the thoracic aorta of a human body, wherein the delivery device (4) comprises a sleeve (42), having at least one lumen for receiving a stent-prosthesis (1), wherein in said lumen an attachment device (44) being movable in the longitudinal direction of the sleeve (42) is provided, wherein the attachment device (44) is designed to be firmly attached to one end of the stent-prosthesis, which end is free from spring elements and is only formed of the flexible material of the tube.

13. A delivery device as claimed in claim 12, wherein the longitudinally movable attachment device (44) is a pull device.

14. A delivery device as claimed in claim 12 or 13 further comprising a balloon (43).

15. A delivery device as claimed in claim 12, 13 or 14 wherein the attachment device (44) has an aperture through which the balloon (43) can be moved, when the balloon is in a deflated state.

16. A delivery set for delivering an stent-prosthesis to the lumen of a blood vessel comprising at least one catheter (42) having at least one lumen and a stent-prosthesis (1) being accommodated in the lumen, the stent-prosthesis (1) having a first section (3) having spring elements (11, 11', 11") at its distal end (122) and a second section (2) being free from spring elements (11, 11', 11") at its proximal end (121), wherein the second section (2) is everted into the first section (3), with the proximal end (121) of the stent-prosthesis (1) being accessible from the proximal end of the first section (3) and being firmly attached to an attachment device (44) being provided in the lumen of the catheter (42).
